# EUROPEAN PATENT SPECIFICATION

(11) **EP 3 198 020 B1**
(45) Date of publication and mention of the grant of the patent: **04.09.2019**
(21) Application number: 15767178.5
(22) Date of filing: 24.09.2015
(51) Int. Cl.: C12Q 1/02, G01N 33/02, G01N 33/569

(54) **METHOD AND KIT FOR DETECTING CONTAMINATION IN PRODUCTS FOR HUMAN UPTAKE**
VERFAHREN UND KIT ZUR DETEKTION DER VERUNREINIGUNG IN PRODUKTEN ZUR AUFNAHME DURCH DEN MENSCHEN
PROCÉDÉ ET KIT DE DÉTECTION D'UNE CONTAMINATION DANS DES PRODUITS DESTINÉS À L'ABSORPTION HUMAINE

(30) Priority: 24.09.2014 EP 14186257
(43) Date of publication of application: 02.08.2017
(73) Proprietor: Frettlöh, Martin, 57080 Siegen (DE)
(72) Inventor: Frettlöh, Martin, 57080 Siegen (DE)
(74) Representative: Fuchs Patentanwälte Partnerschaft mbB
(86) International application number: PCT/EP2015/071992
(87) International publication number: WO 2016/046320

(56) References cited:
- US-A1- 2007 072 174
- O. M. CLOAK ET AL: "Quorum Sensing and Production of Autoinducer-2 in Campylobacter spp., Escherichia coli O157:H7, and Salmonella enterica Serovar Typhimurium in Foods", APPLIED AND ENVIRONMENTAL MICROBIOLOGY, vol. 68, no. 9, 1 September 2002 (2002-09-01), pages 4666-4671, XP055170855, ISSN: 0099-2240, DOI: 10.1128/AEM.68.9.4666-4671.2002
- LINGENG LU ET AL: "Autoinducer-2-Like Activity on Vegetable Produce and Its Potential Involvement in Bacterial Biofilm Formation on Tomatoes", FOODBORNE PATHOGENS AND DISEASE, vol. 2, no. 3, 1 September 2005 (2005-09-01), pages 242-249, XP055170828, ISSN: 1535-3141, DOI: 10.1089/fpd.2005.2.242
- Anonymous: "Vibrioharveyi (ATCC BAA-1117) Product Sheet", ATCC , 2013, XP002736220, Retrieved from the Internet: URL:www.atcc.org/~/ps/BAA-1117.ashx [retrieved on 2015-02-20]
- A. JAMUNA BAI ET AL: "Bacterial Quorum Sensing and Food Industry", COMPREHENSIVE REVIEWS IN FOOD SCIENCE AND FOOD SAFETY, vol. 10, no. 3, 6 May 2011 (2011-05-06), pages 183-193, XP055170862, ISSN: 1541-4337, DOI: 10.1111/j.1541-4337.2011.00150.x

## Description

Uptake of products that are contaminated with pathogens or toxins may cause diseases in humans. Therefore, high standards of hygiene need to be fulfilled during the production of respective products. Especially high criteria of hygiene need to be met for the production of eye drops and even more for injection solutions. But also products for oral uptake like drugs for oral application or also food should not be contaminated in order for the products to be harmless to health. The same applies for products made for uptake by external application on skin like creams or ointments because disease may potentially even be caused by contact with the skin depending on the contamination being present in the product.

Despite high standards of hygiene it is possible that products for human uptake are contaminated with pathogens or toxins. Therefore, such products need to be routinely tested for contamination so that contaminated products can be identified and excluded from being placed on the market. This is especially the case for products that generally meet less strict criteria of hygiene during the production process so that the chances of products being contaminated are higher. Especially food bears a high risk of being contaminated because the process of food production often begins with raw material originating from living organism like animals or plants, which are more difficult to keep under controlled conditions as compared to inorganic raw material and which inherently are prone to contamination with pathogens and/or toxins. Moreover, contamination may occur at various stages of the food supply chain, for example at the sites of food production, i.e. for example at farms, at the sites of food processing, i.e. for example at slaughterhouses, at the sites of food distribution, i.e. for example at warehouses or trucks, at the sites of local suppliers, i.e. for example at supermarkets and also at the sites of consumption, i.e. for example in restaurants or in the customer's kitchen, because in all such sites the standards of hygiene are much lower than in the laboratories and factories of the pharmaceutical and cosmetic industry.

Thus, in principle food samples have to be tested at various sites throughout the food supply chain in order to avoid diseases caused by contaminated food. However, the current methods for detecting contamination are complicated and require advanced laboratory equipment and technically trained personnel. Consequently, the current tests cannot be done locally at the sites where the samples are taken but instead the samples have to be transported to specialized laboratories for detecting contamination in food. This process is cost-intensive and time-consuming. Furthermore, the current test methods are time-consuming themselves independent of the time required for sample transport.

For example *Salmonellae,* which represent one of the main food contaminations, are currently either detected by bacteriological culturing methods on the one hand, or by polymerase chain reaction (PCR), by Enzyme Linked Immunosorbent Assay (ELISA) or by comparable test systems on the other hand. With PCR, ELISA or comparable test systems the results are available faster as compared to bacteriological culturing methods, but these kinds of tests do not distinguish between living or dead *Salmonellae* and therefore the results are not really of high value when a decision has to be made in the production process.

While a result from PCR may not be expected before 36 to 48 hours after sample processing was initiated in the specialized laboratory, the bacteriological culturing methods require even a time span of 3 to 5 days before a result may be obtained. Consequently, using the current methods the results are often obtained so late that contaminated food has already been placed on the market so that *Salmonellae* cause a multitude of severe infections every year.

Moreover, before contaminated food has been identified it is impossible to isolate the respective food from its surrounding so that uncontaminated food may become contaminated as long as the test results are not available. Thus, the longer it takes to identify contaminated food, the more the contamination will generally spread. The current test methods are also expensive. Therefore, the number of samples taken is very limited today for economical reasons resulting in even less pathogen contamination of food to be detected.

Even though there are generally higher standards of hygiene for the production of pharmaceutical and cosmetic products compared to the production of food, contamination of such products cannot be excluded per se and samples have to be tested for possible contamination as well. The tests applied in these sectors are basically the same as applied in the food sector as no better methods are available today.

It would thus be preferable to detect molecules that are natively present in samples so that complex sample processing can be avoided. It has been known that several bacterial species communicate by a mechanism termed quorum sensing that includes secretion of communication molecules, for example different kinds of autoinducer molecules. However, detection of such communication molecules has not been considered a feasible approach for detecting contamination in products for human uptake for several reasons. First, there have been doubts about the positive correlation between the bacterial density and the concentration of autoinducer molecules (see Lu et al. in Foodborne Pathogens and Disease, Vol. 2, No. 3, September 2005, pages 242-249) so that detection of autoinducer molecules was suspected not to deliver reliable quantitative results. Furthermore, results from basic research with regard to detection of autoinducer molecules in liquid foods were not promising as well. Cloak et al. (in Applied and Environmental Microbiology, Vol. 68, No. 9, September 2002, pages 4666-4671) found that no autoinducer was detectable in apple juice after inoculation and incubation for 1 or 2 days at different temperatures using a fluorescence-based bioreporter assay. The results were also poor with regard to autoinducer detection in milk as compared to classic brucella broth medium. Autoinducer was detectable in chicken broth, however, in reasonable amounts only after incubation for 24 hours at elevated temperatures. It is unclear what the reasons for the unpromising results were. One reason might be that Cloak et al. used cell-free supernatants as samples, which was supposed to be the gold standard up to now. However, the inventors of the present invention found that better results can be obtained if samples are not subjected to complicated clarification steps like centrifugation or filtration. Whatever the reason might be, it is clear that the results of Cloak et al. suggested that quorum sensing systems might indeed be targets for the development of novel antibacterial agents, however, detection of autoinducer molecules was not a promising approach for detection of pathogens in foods.

A way of detecting bacterial contamination in food samples has been described in US 2007/0072174 A1. According to this approach, bacteria to be detected in food samples are infected by specific phages that induce production of autoinducer molecules that are detected by bioreporter cells that generate an optical signal upon contact with autoinducer molecules. Thus, the autoinducer molecules are typically not present in the food samples but have to be induced by phage infection in order for the method to work. It turned out that the proposed method has not gained acceptance in practice.

The main reason seems to be that a complex and time-consuming processing of food samples is necessary for induction of autoinducer production. In addition, the induction step is error-prone and failure of induction for technical reasons might easily be misinterpreted as absence of contamination.

It is therefore an object of the present invention to provide a method for detecting contamination in products for human uptake that overcomes the disadvantages of the prior art.

The object is solved by the method of the patent claims. Autoinducer is used as marker for bacteria.

The object is especially solved by a method for detecting contamination in products, in particular products for human uptake, comprising the steps of
A. Providing a sample, in particular a solid sample or non-clarified liquid sample,
B. Providing detector cells, wherein the detector cells comprise at least one contamination receptor, wherein the contamination receptor is a polypeptide,
C. Providing a detection medium comprising the sample and the detector cells,
D. Detecting a signal generated in the detection medium, wherein the generation of the signal is dependent on an interaction of the contamination receptor of the detector cells with a contamination-associated molecule that is present in the product in case of contamination, and
E. Analyzing the signal,
   wherein the sample is neither filtered nor centrifuged to remove cells before detecting the signal, wherein the signal is an optical signal, wherein the contamination-associated molecule is selected from the group consisting of autoinducer-1 (AI-1), autoinducer-2 (AI-2), autoinducer-3 (AI-3) and mixtures of two or more thereof, and wherein the detector cells are bacteria. Preferably, the sample is subjected to no clarification step whatsoever.

The generation of the signal is dependent on an interaction of the contamination receptor of the detector cells with a contamination-associated molecule that is present in the product in case of contamination. In other words the contamination-associated molecule is not generated in the detection medium or the sample but is the entity within the product that is indicative of contamination.

The method of the present invention can be suitably used for detecting contamination in any products for human uptake. Preferably, the products for human uptake are selected from the group consisting of liquid products, solid products and mixtures thereof. Particularly preferably, the products are selected from the group consisting of liquid products and solid products. Even more preferably, the products are solid products.

Preferably, the products for human uptake are selected from the group consisting of pharmaceutical products, cosmetic products and food. More preferably, the products are selected from the group consisting of pharmaceutical products and food. Even more preferably, the products are food. The product within which contamination is detected can be a living organism. The method of this invention can also be used to detect bacteria in the intestine or body fluids or tissue of a human or other animal, or to detect infections in an animal. The method may also be used to detect contamination in specimen that is taken from the environment (e.g. plants, soil).

According to the present invention, pharmaceutical products are preferably understood as any products intended for internal or external use in the medical diagnosis, cure, treatment or prevention of human disease. Importantly, the present method can preferably be used for detecting contamination in any pharmaceutical product independent of the active agent and other ingredients being present in the respective product. The reason is that the present invention is directed to certain contaminations in the product and not to the specific regular ingredients of the product. According to the present invention, pharmaceutical products may preferably be tested for contamination using the method of the present invention independent of the dosage form of the respective products.

According to the present invention, cosmetic products are preferably understood as any products used to enhance the appearance or odor of the human body. Importantly, the present method can preferably be used for detecting contamination in any cosmetic product independent of the ingredients being present in the respective product. The reason is that the present invention is directed to certain contaminations in the product and not to the specific regular ingredients of the product.

According to the present invention, food is preferably understood as any substance consumed to provide nutritional support for the body. Importantly, the method of the present invention can preferably be used to detect contamination in both liquid foods and solid foods.

According to the present invention, liquid foods are preferably selected from the group consisting of plant-based liquid foods, animal-based liquid foods, inorganic liquid foods and mixtures thereof. Preferred plant-based liquid foods are selected from the group consisting of lemonades, teas and juices. More preferably, the plant-based liquid food is juice. Preferred animal-based liquid foods are selected from the group consisting of eggs and milk. Preferred inorganic liquid food is water.

According to the present invention, solid foods are preferably selected from the group consisting of plant-based solid foods, animal-based solid foods, fungi-based solid foods, bacteria-based solid foods, inorganic solid foods and mixtures thereof. Preferred plant-based solid foods are selected from the group consisting of vegetables and fruits. Preferred animal-based foods are selected from the group consisting of fish and meat. More preferably, the animal-based solid food is meat.

The method of the present invention can be used for detecting contamination in products for human uptake. Preferably, the contamination is a pathogen.

According to the present invention, the pathogen is preferably selected from the group consisting of viruses, prokaryotic cells and eukaryotic cells. Preferably, the pathogen is selected from prokaryotic cells. Even more preferably, the pathogens are bacteria. Even more preferably, the pathogens are selected from the group of gram-negative bacteria. Even more preferably, the pathogens are selected from the group of *Salmonella* spp.

Nucleotides are preferably selected from the group consisting of mononucleotides, oligonucleotides, polynucleotides and mixtures thereof. According to the present invention, oligonucleotides are preferably nucleotides comprising from two to ten mononucleotides. Polynucleotides are preferably nucleotides comprising at least eleven mononucleotides.

Saccharides are preferably selected from the group consisting of monosaccharides, oligosaccharides, polysaccharides and mixtures thereof. According to the present invention, oligosaccharides are preferably saccharides comprising from two to ten monosaccharides. Polysaccharides are preferably saccharides comprising at least eleven monosaccharides.

Peptides are preferably selected from the group consisting of oligopeptides and polypeptides. According to the present invention, oligopeptides are preferably peptides comprising from two to ten amino acids and polypeptides are preferably peptides comprising at least eleven amino acids. The terms "polypeptide" and "protein" will be used interchangeably in the present description.

According to the present invention, small molecules are preferably organic molecules other than nucleotides, peptides and saccharides. Preferably, small molecules have a molecular weight of at most 2 kDa, more preferably at most 1 kDa.

The method of the present invention requires that a sample is provided according to step A. Preferably, the sample is the product for human uptake to be tested for contamination or a part of the respective product. Preferably, the sample may be selected from the group consisting of liquid samples, solid samples and mixtures thereof.

In case of solid products, the solid product or at least a part thereof may preferably be used as solid sample.

Liquid samples may preferably be prepared from products for human uptake selected from the group consisting of liquid products, solid products and mixtures thereof. In preferred embodiment the sample may be taken from pipes, duct or conduit that are used for processing or dispensing a product, in particular a product for human uptake. The sample may preferably be taken by contacting the pipe, duct or conduit with a washing liquid to obtain a non-clarified liquid sample. The sample may then be used in the method of this invention without any clarification steps. Alternatively, the pipe, duct or conduit can be wiped with a transfer implement such as a cloth, sponge or other material that is suitable to transfer any contamination and/or contamination associated molecule from the pipe, duct or conduit to the transfer implement. The transfer implement may be used as a solid sample. Exemplary pipes, ducts and conduits are widespread in the food processing industry. In case of liquid products, the liquid products or parts thereof may be directly used as liquid samples. Alternatively, the liquid products may be diluted by using a dilution liquid in order to provide the liquid sample.

In case of solid products, a liquid sample may preferably be prepared from the solid product by contacting at least a part of the solid product with an extraction liquid. The extraction liquid may preferably be used as liquid sample after being in contact with the solid product. Preferably, it is not important how the solid product is contacted with the extraction liquid as long as it is ensured that a sufficient amount of the contamination-associated molecules described below may potentially be present in the liquid sample that is generated by contacting the solid product with the extraction liquid. Preferably, the solid product is contacted with the extraction liquid by dousing the solid product in the extraction liquid. Particularly preferably, the solid product is contacted with the extraction liquid by incubating at least a part of the solid product together with the extraction liquid in an extraction container. Alternatively or in addition, the solid product may be contacted with the extraction liquid by dousing the solid product with the extraction liquid. The solid product may also be wiped with the extraction liquid in order to prepare a liquid sample from the solid product.

If the product for human uptake to be tested for contamination is animal-based food, contamination of the product may occur because the animal, from which the product is derived, is infected with pathogens. Consequently, in case of animal-based food, it is desirable that the sample may be taken directly from the respective animal during or even before processing of the animal-based food in order to identify the source of contamination as early as possible within the food supply chain and to avoid contamination spreading.

According to the present invention, the sample may be preferably derived from animals during or even before processing of the animal-based food. Particularly preferably, samples may be provided from living animals. Thus, for example body fluids of animals may be used as liquid samples according to the present invention. Preferred body fluids are selected from the group consisting of blood, saliva and urine. More preferably, the body fluid is blood. Alternatively, the sample may also be derived from the animal by contacting the animal with an extraction liquid as described above for the preparation of liquid samples from solid products. In other embodiments, animal's feces may be used as solid sample. Alternatively, a liquid sample may be provided by contacting the animal's feces with an extraction liquid as described above.

According to the present invention, liquid samples are non-clarified samples. A liquid sample is called "clarified sample", if the respective liquid sample was subjected to one or more clarification steps after the liquid sample was prepared as described above. A "non-clarified sample" is a liquid sample in which any cells that may have been present in the original sample are still present in the sample when the detection medium is prepared under step C of the present method. In particular, such a sample was not subjected to any clarification step.

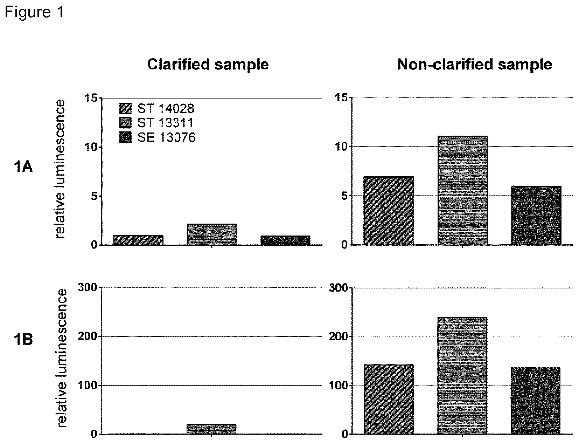

Surprisingly it has been found by the inventors that a better detection result can be obtained with non-clarified samples as compared to clarified samples. Therefore, the liquid sample is a non-clarified sample according to the present invention. It has surprisingly been found that using non-clarified samples leads to improved results (see Example 1, Fig.1). Non-clarified samples are used, including solid samples and liquid samples.

The method of the present invention requires that detector cells are provided according to step B. The detector cells preferably facilitate the generation of the signal.

The detector cells are bacteria. Preferably, the detector cells are selected from the group consisting of gram-positive bacteria and gram-negative bacteria. More preferably, the detector cells are selected from the group of gram-negative bacteria. Even more preferably, the detector cells are selected from the group of *Vibrio* spp.. Even more preferably, the detector cells belong to the species of *Vibrio harveyi.* Even more preferably, the detector cells are selected from the *Vibrio harveyi* strains BB-170 (ATCC number BAA-1117) and MM32 (ATCC number BAA-1121).

The detector cells may be provided dispersed in a liquid detector cell buffer as a detector cell dispersion. Preferably, the detector cell dispersion is liquid. Preferably, the liquid detector cell buffer is aqueous. Preferably, the liquid detector cell buffer is selected from the group consisting of lysogeny broth medium (LB medium) and autoinducer bioassay medium (AB medium). More preferably, the liquid detector cell buffer is AB medium. Both, LB medium and AB medium are well known to the skilled person. Preferably, LB medium comprises 10 g/l tryptone, 5 g/l yeast extract and 10 g/l NaCl in distilled water. Preferably, AB medium comprises 17.53 g/l NaCl, 6.02 g/l MgSO₄, 2 g/l vitamin-free casamino acids, 10 mM potassium phosphate, 1 mM L-arginine and 1% by volume glycerol in distilled water. In preferred embodiments, the liquid detector cell buffer comprises additionally boric acid. Boric acid increases the sensitivity of the method of the present invention if it is used in suitable amounts. Preferably, the liquid detector cell buffer comprises boric acid in an amount of at least 0.01 mM, more preferably at least 0.05 mM, more preferably at least 0.1 mM, more preferably at least 0.2 mM. The amount of boric acid should not be too high. Preferably, the liquid detector cell buffer comprises boric acid in an amount of at most 2 mM, more preferably at most 1 mM, more preferably at most 0.5 mM, more preferably at most 0.3 mM. Most preferably, the detector cell buffer comprises boric acid in an amount of about 0.25 mM.

In alternative embodiments, the detector cells may also be provided in solid form. In these embodiments, the detector cells are preferably provided as lyophilisate or as a glycerol stock. Alternatively, the detector cells may also be provided within a semisolid matrix.

According to the present invention, the detector cells preferably comprise at least one contamination receptor suitable for detecting a contamination-associated molecule. Preferably, the contamination receptor is at least partially located at the surface of the detector cells so that the contamination receptor has direct access to the surrounding. Thereby, detection of contamination-associated molecules that are outside the detector cells is enabled. However, alternatively or in addition, the detector cells may comprise a contamination receptor that is located entirely inside the detector cells so that contamination-associated molecules that enter the detector cells might be detected. In such embodiments, the detector cells preferably comprise at least one channel that allows specific or unspecific transport of the contamination-associated molecules into the detector cells. A channel that allows specific transport is preferred. Preferably, the contamination-associated molecule is not produced by the detector cells. Otherwise, a false positive signal may be obtained.

The contamination receptor is a polypeptide. Polypeptides might include various post-translational modifications. Suitable post-translational modifications are known to the skilled person. Preferable post-translational modifications comprise phosphorylations and glycosylations. In embodiments, in which the contamination receptor is at least partially located at the cell surface, the contamination receptor is preferably selected from the group of transmembrane proteins.

Preferably, the contamination receptor is suitable for detecting contamination-associated molecules. Preferably, the interaction of the contamination receptor with the contamination-associated molecules is characterized by a dissociation constant of at most 10⁻⁵ mol/l, more preferably at most 10⁻⁶ mol/l, more preferably at most 10⁻⁷ mol/l, more preferably 10⁻⁸ mol/l, more preferably at most 10⁻⁹ mol/l and even more preferably at most 10⁻¹⁰ mol/l. A low dissociation constant enables a specific interaction of the contamination receptor with the contamination-associated molecule. The dissociation can be determined by various methods well known to the skilled person. Preferably, the dissociation constant is determined by using surface plasmon resonance. Preferably, the dissociation constant is determined at a temperature of from 20°C to 30°C, more preferably at a temperature of about 25°C. Preferably, the dissociation constant is determined at a pH of from 7 to 8, more preferably at a pH of about 7.4.

The contamination-associated molecules according to the present invention are selected from the group consisting of autoinducer-1 (AI-1), autoinducer-2 (AI-2), autoinducer-3 (AI-3) and mixtures of two or more thereof. More preferably, the contamination-associated molecules are selected from the group consisting of AI-1, AI-2 and mixtures thereof. Even more preferably, the contamination-associated molecule is AI-2.

According to the present invention, the contamination-associated molecule is present in the products for human uptake in case of contamination. Otherwise, i.e. if the contamination-associated molecule was not present in the products for human uptake in case of contamination, induction of production of the contamination-associated molecule would be required in order for a sample to be used in the method of the present invention. Such additional processing of the sample prior to using the sample in the method of the present invention would not only be time-consuming but also error-prone. Importantly, failure of induction might easily be misinterpreted as absence of contamination. Moreover, induction of production of the contamination-associated molecule is only possible if the contamination is a pathogen. Poisons and other contaminations could not be detected, if they were not present in the products for human uptake in case of contamination. Finally, even if the contamination was a pathogen, induction of production of the contamination-associated molecule after taking the sample would only be possible, if the pathogen would also be present in the sample. In contrast, according to the method of the present invention, in which the contamination-associated molecules are present in the products for human uptake if the product is contaminated, detecting the contamination is not dependent on the presence of a pathogen in the sample. Thus, using the method of the present invention, also contamination of products for human uptake resulting from biofilm formation, for example in pipes containing liquid products for human uptake, can be detected because contamination-associated molecules are present in the liquid due to secretion in contrast to the pathogens themselves that do not enter the liquid but rather stay in the biofilm.

According to a preferred embodiment of the present invention, two or more contamination-associated molecules are examined. By examining two or more contamination-associated molecules, the contamination can preferably be detected more specifically as compared to embodiments in which only one contamination-associated molecule is examined. However, increasing the number of examined contamination-associated molecules is also connected with increased complexity. Thus, it is particularly preferable that exactly two contamination-associated molecules are examined. Preferably, both Al-2 and Al-3 are examined. If the examination of Al-2 and Al-3 reveals that both Al-2 and Al-3 are present, this is an especially strong indication that the contamination is *Salmonellae.*

The method of the present invention requires that a detection medium is provided which comprises the sample and the detector cells according to step C. Preferably, the detection medium is semisolid or liquid. More preferably, the detection medium is liquid. The provided detection medium has preferably a volume of at least 10 µl, more preferably at least 20 µl, more preferably at least 50 µl, more preferably at least 80 µl. If the volume is too low, detection of the signal is compromised. The provided detection medium has preferably a volume of at most 5 ml, more preferably at most 2 ml, more preferably at most 1 ml, more preferably at most 500 µl, more preferably at most 200 µl, more preferably at most 150 µl. If the volume is too large, the detection medium may be too diluted resulting in reduced signal generation.

Importantly, the method of the present invention may be performed with both solid samples and liquid samples.

In case of solid samples, the detection medium may preferably be provided by mixing the solid sample with the detector cell dispersion.

In case of liquid samples, the detection medium may preferably be provided by mixing the detector cells with the liquid sample. Before mixing with the liquid sample, the detector cells may be provided as detector cell dispersion. In such embodiments, the volume ratio of liquid sample to detector cell dispersion is preferably at least 1:20, more preferably at least 1:15, more preferably at least 1:12, more preferably at least 1:10. If the ratio is too low, the signal generation will be compromised. Preferably, the volume ratio of liquid sample to detector cell dispersion is at most 1:2, more preferably at most 1:5, more preferably at most 1:8. If the ratio is too large, the signal generation will be compromised. Ratios chosen accordingly result in optimal signal generation. Particularly preferably, the volume ratio of liquid sample to detector cell dispersion is about 1:9. Alternatively, the detector cells may be provided in solid form, for example as a lyophilisate or as a glycerol stock and mixed as such with the liquid sample in order to provide the detection medium. In an alternative embodiment, the detector cells may be provided within a semisolid matrix.

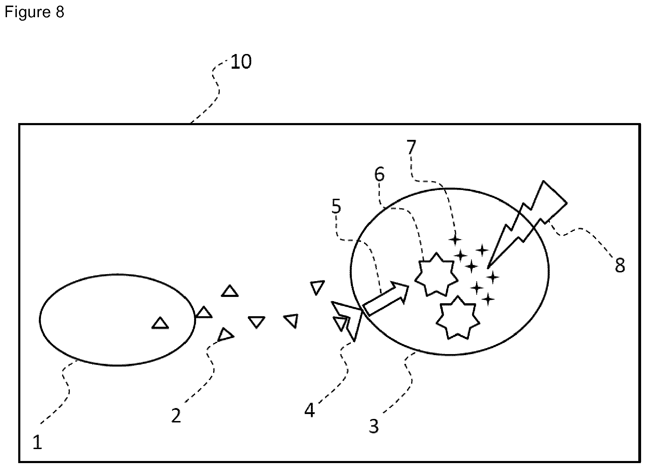

The method of the present invention requires that a signal generated in the detection medium is detected according to step D. Signal generation in the detection medium according to an exemplary embodiment of the present invention is shown schematically in Figure 8.

Preferably, the provided detection medium is incubated for a time interval of at least 10 minutes, more preferably at least 30 minutes, more preferably at least 1 hours, more preferably at least 1.5 hours, more preferably at least 2 hours, even more preferably at least 3 hours prior to detection of the signal. This ensures that a sufficiently strong signal is generated. Preferably, the provided detection medium is incubated for a time interval of at most 24 hours, more preferably at most 12 hours, even more preferably at most 10 hours more preferably at most 8 hours, more preferably at most 6 hours, even more preferably at most 5 hours prior to detection of the signal. If the detection medium is incubated too long, an increased signal may be obtained in the detection medium comprising the negative control sample described below. The incubation is preferably done at a temperature of from 10°C to 45°C, more preferably from 15°C to 40°C, more preferably from 20°C to 37°C, more preferably from 25°C to 35°C, more preferably from 28°C to 32°C, even more preferably about 30°C.

The signal generated in the detection medium is an optical signal. More preferably, the signal is electromagnetic radiation emitted from the detection medium. Preferably, the emitted electromagnetic radiation has its emission maximum in a wavelength range of from 200 nm to 1100 nm, more preferably from 250 nm to 1000 nm. Particularly preferably, the emitted electromagnetic radiation has its emission maximum in the range of visible light. Preferably, the emitted electromagnetic radiation has its emission maximum in a wavelength range of from 380 nm to 800 nm, more preferably from 390 nm to 700 nm, even more preferably from 420 nm to 680 nm.

Preferably, the signal is detected with a detector. Preferably, the detector is a photodetector. Suitable detectors are well known to the skilled person. Suitable detectors may for example be charge-coupled devices (CCDs), photodiodes and photomultipliers. Preferably, the detector is used as single-photon detector (SPD). An SPD enables detection and counting of individual photons that reach the detector. Preferably, the detector is configured for detection of light with a wavelength corresponding to the emission maximum of the signal. Preferably, light having a wavelength that differs from the wavelength of the emission maximum of the signal by more than 100 nm, more preferably by more than 50 nm, even more preferably by more than 10 nm, is not detected by the detector.

According to the present invention, the signal is generated in the detection medium, for example inside or outside the detector cells. Preferably, the intensity of the signal is dependent on an interaction of the contamination receptor of the detector cells with a contamination-associated molecule. The contamination-associated molecule may be modified inside the detector cells prior to interaction with the contamination receptor. Particularly, the contamination-associated molecule may be phosphorylated prior to interaction with the contamination receptor. However, the contamination receptor may also interact with unmodified contamination-associated molecules. Preferably, the interaction of the contamination receptor with the contamination-associated molecule induces a signal-transduction cascade in the detector cells. Preferably, the signal-transduction cascade results in altered transcription of at least one detector gene present in the detector cells. Preferably, the altered transcription is increased gene expression.

Preferably, the interaction of the contamination receptor with the contamination-associated molecule results in altered levels of at least one detector molecule especially inside and/or outside the detector cell. Preferably, the detector molecule is produced by the detector cells. Preferably, altered levels are increased levels. Preferably, the detector molecule is selected from the group consisting of small molecules, nucleotides, peptides, saccharides and mixtures thereof. More preferably, the detector molecule is a polypeptide. Preferably, the polypeptide is selected from the group consisting of monomeric and oligomeric proteins. Preferably, the oligomeric proteins are dimers.

Preferably, the detector molecule is selected from the group consisting of enzymes, fluorescent proteins and chromogenic proteins. More preferably the detector molecule is selected from the group consisting of enzymes and fluorescent proteins. Even more preferably, the detector molecule is an enzyme.

Fluorescent proteins used in the present invention are proteins that exhibit fluorescence upon exposure to suitable electromagnetic excitation radiation. A preferred fluorescent protein is green fluorescent protein (GFP). Enzymes used in the present invention are molecules that accelerate the rate of specific chemical reactions.

Thus, enzymes act as catalysts. Preferably, enzymes are selected from the group consisting of β-galactosidase and luciferase proteins. Even more preferred, the enzyme is selected from luciferase proteins.

If the detector molecule is a protein, the detector gene preferably encodes the respective protein.

Preferably, the signal is generated by a signal molecule. Preferably the signal molecule emits electromagnetic radiation with emission properties as described above.

If the detector molecule is fluorescent, the detector molecule preferably is the signal molecule according to the present invention. In such embodiments, signal generation in the detection medium is preferably elicited by excitation with electromagnetic radiation suitable for exciting the fluorescent detector molecule. The skilled person is well aware of plenty of possibilities of exciting fluorescent molecules. According to the present invention, the fluorescent detector molecule is preferably excited with electromagnetic radiation by use of an excitation source. Various suitable excitation light sources are known to the skilled person. Preferably, the excitation source is selected from the group consisting of lasers, LEDs and lamps. Preferable lamps are selected from the group consisting of xenon arcs and mercury-vapor lamps.

Preferably, the signal molecule is not the detector molecule. Preferably, altered levels of the detector molecule result in altered levels of the signal molecule especially inside and/or outside the detector cell. In preferred embodiments, the detector molecule is a luciferase protein. In these embodiments, the signal molecule preferably is an oxyluciferin. Preferably, the oxyluciferin is produced by oxidation of luciferin catalyzed by the luciferase protein. The luciferin may be added to the detection medium. Preferably, the luciferin is reduced flavin mononucleotide (FMNH₂).

The method of the present invention requires that the signal is analyzed according to step E. Signal analysis preferably comprises the steps of signal quantification and normalization of the quantified signal. Preferably, the signal is normalized to one or more quantified signals obtained from control samples.

Preferably, the signal generated in the detection medium is quantified after detection in order to obtain a quantified signal. In a preferred embodiment, the detected photons are counted and the quantified signal is obtained as counted photons per time interval, for example counted photons per minute (cpm).

Preferably, the quantified signal obtained from a sample of a product for human uptake to be tested for contamination (test sample) is normalized to a quantified signal obtained from a control sample. Preferably, the quantified signal from the control sample is obtained by subjecting the control sample to the method of the present invention in the same way as the test sample. Preferably, the control sample is a sample of known contamination state, i.e. it is known whether the respective sample corresponds to contaminated products (positive control sample) or uncontaminated products (negative control sample). Preferably, the negative control sample is selected from the group consisting of extraction liquid that was not in contact with a product for human uptake, dilution liquid that was not in contact with a product for human uptake, detector cell buffer and mixtures thereof. Preferably, a normalized signal is obtained by building the ratio of the quantified signals obtained from the test sample and the control sample. Preferably, the respective ratio indicates whether the tested product is contaminated. Preferably, a ratio of at least 200, more preferably at least 100, more preferably at least 50, more preferably at least 10, even more preferably at least 5 indicates that the product is contaminated.

Preferably, a result whether or not a product for human uptake is contaminated may be obtained by using the method of the present invention within a time frame of at most 4 hours, more preferably at most 3 hours, more preferably at most 2 hours, more preferably at most 1 hour, even more preferably at most 30 minutes, even more preferably at most 15 minutes as calculated starting from the provision of the sample.

### Examples

### Example 1: Comparison of clarified and non-clarified samples

In order to test detection of contamination by the method of the present invention, cultures of three different pathogens, namely *Salmonella* Typhimurium 14028, *Salmonella* Typhimurium 13311 and *Salmonella* Enteritidis 13076, were prepared in LB medium. Samples taken from the respective cultures were used as non-clarified samples. Clarified samples were prepared by subjecting samples to two subsequent clarification steps. First, the samples were centrifuged with an acceleration of 6000 x g for 5 minutes at room temperature (about 20 to 25°C) in order to pellet the *Salmonella* cells. The supernatant was then filtered using a filter with a pore size of 0.2 µm in order to remove any *Salmonella* cells that might have been present in the supernatant. The resultant filtrate was used as clarified sample.

Cells from the *Vibrio harveyi* strain BB-170 were used as detector cells. Cells of the respective strain produce a luciferase protein upon exposure to AI-2 so that a signal is generated in detection medium comprising BB-170 cells and an AI-2 comprising sample as well as FMNH₂ as a suitable luciferin.

The detector cells were provided as detector cell dispersion by inoculating liquid detector cell buffer with detector cells taken from a glycerol stock, incubating the detector cells for about 16 hours in the detector cell buffer to provide an overnight culture and diluting the overnight culture 1:5000 with fresh liquid detector cell buffer. AB medium was used as detector cell buffer.

The detection medium of the present invention was prepared by mixing 90 µl of the detector cell dispersion with 10 µl sample (either clarified or non-clarified as described above). The detection medium comprising the sample and the detector cells was incubated for 2 hours (Figure 1A) or 3 hours (Figure 1B) at a temperature of 30°C in a microtiter plate. Then the signal generated in the detection medium was detected at a wavelength of from 400 nm to 700 nm for a time interval of 1000 to 2000 ms using a microplate reader from Tecan. The signal was quantified by counting the detected photons. A normalized signal was obtained by building the ratio of the quantified signals obtained from the test sample and the negative control sample. LB medium without *Salmonellae* was used as negative control sample.

After 2 hours of incubation, the signal generated in detection medium comprising a clarified sample was in the range of the negative control. In contrast, the signal generated in detection medium comprising a non-clarified sample was approximately five to ten times higher than the signal generated in detection medium comprising the negative control sample. Thus, detection of *Salmonellae* is enabled by the method of the present invention.

After 3 hours of incubation, the signal generated in detection medium comprising a clarified sample of S. Typhimurium 13311 was approximately 20 times higher than the signal generated in detection medium comprising the negative control sample. In contrast, the signal generated in detection medium comprising clarified samples from S. Typhimurium 14028 and S. Enteritidis 13076, respectively, was not higher than the signal generated in detection medium comprising the negative control sample. Thus, using the clarified samples, the pathogen was only detected in samples of S. Typhimurium 13311.

After 3 hours of incubation, the signal generated in detection medium comprising a non-clarified sample of S. Typhimurium 13311 was more than 200 times higher than the signal generated in detection medium comprising the negative control sample. Moreover, even the signal generated in detection medium comprising non-clarified samples from S. Typhimurium 14028 and S. Enteritidis 13076, respectively, was more than 100 times higher than the signal generated in detection medium comprising the negative control sample. Thus, using the non-clarified samples, the pathogen was detected in all samples tested. Furthermore, the normalized signal was more than ten times higher in the case of non-clarified samples of S. Typhimurium 13311 as compared to clarified samples of S. Typhimurium 13311 further indicating that more robust results can be obtained from non-clarified samples.

### Example 2: Detection of seven different Salmonella serovars

In order to test whether the method of the present invention was suitable for detecting different *Salmonella* serovars, the method was applied to non-clarified samples taken from cultures of seven different *Salmonella* serovars prepared as described in Example 1. In addition to samples from cultures of S. Typhimurium 13311, S. Typhimurium 14028 and S. Enteritidis 13076, samples from cultures of S. Typhimurium Isolat, S. Gruppe D1, S. Infantis and S. Derby were tested. Cells from the *Vibrio harveyi* strain BB-170 were used as detector cells as described above.

In order to test whether the method of the present invention enables discrimination between the pathogen to be detected (in this case *Salmonellae*) and other bacteria that are potentially present in a sample and that do not produce AI-2, a non-clarified sample from a culture of the *Escherichia coli* strain DH5α was tested using the method of the present invention. Importantly, it was expected that the signal generated in the detection medium comprising the sample of DH5α cells was low because DH5α cells are known to not produce AI-2.

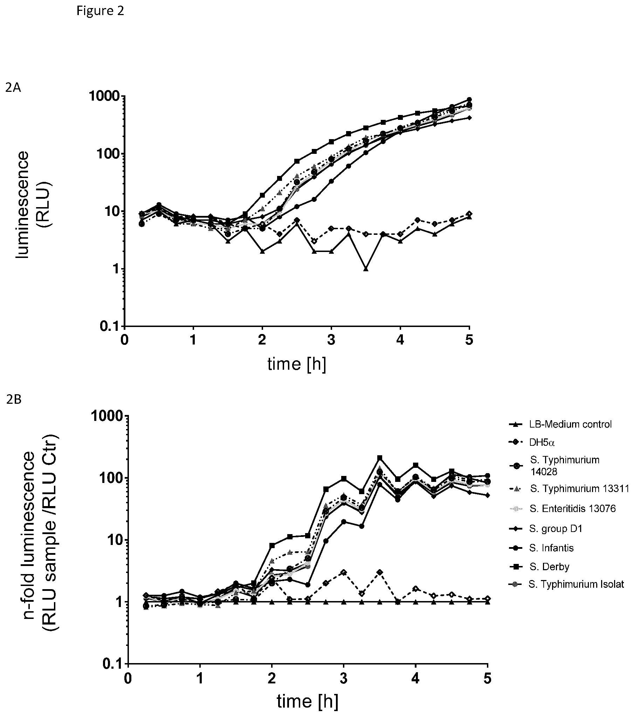

The results of both experiments are summarized in Figure 2. The signal generated in the detection medium was detected at the indicated time points as described in Example 1. The quantified signal is presented as relative light units (RLU) in Figure 2A and the normalized signal representing the ratio of the quantified signals obtained from the test sample and from the negative control sample is shown in Figure 2B.

The highest ratios of the quantified signals obtained from the test sample and from the negative control sample was obtained after incubation of the detection medium for a time of from 2 to 5 hours. Prior to 2 hours of incubation the signal in the detection medium comprising the test sample was low.

Looking at the signal detected after 2 to 5 hours of incubation, the signal generated in detection medium comprising the sample of DH5α cells was very low compared to the signal generated in detection medium comprising a sample from one of the *Salmonella* cultures of S. Typhimurium 13311, S. Typhimurium 14028 and S. Enteritidis 13076 known to produce a strong signal (see Example 1). In fact, the DH5α-associated signal was in the range of the negative control (only LB medium as sample) indicating that the method of the present invention is not prone to deliver false positive results in the presence of bacteria that do not produce AI-2 in the sample.

Furthermore, the signal generated in detection medium comprising samples from one of the *Salmonella* cultures of previously untested serovars (S. Typhimurium Isolat, S. Gruppe D1, S. Infantis and S. Derby) was comparable to the signal obtained from samples of the previously tested serovars (S. Typhimurium 13311, S. Typhimurium 14028 and S. Enteritidis 13076, see Example 1). Thus, the method of the present invention enables detection of various different *Salmonella* serovars.

### Example 3: Influence of glucose in the sample on the detection of Salmonella

Many products for human uptake to be tested for contamination according to the present invention may comprise glucose. Consequently, samples taken from such products may potentially comprise glucose as well. Therefore, the influence of glucose on the performance of the method of the present invention was tested.

The method of the present invention was applied on non-clarified samples taken from cultures of the seven different *Salmonella* serovars described in Example 2. A sample comprising only LB medium was used as negative control sample. Also a non-clarified sample of DH5α cells was tested as described in Example 2. In order to test the influence of glucose on the performance of the method of the present invention, the respective experiments were performed twice, once without glucose and once with 0.5% by weight glucose being present in the detection medium.

The generated signal was detected after incubation for 3 hours at a temperature of 30°C in a microtiter plate as described in Example 1.

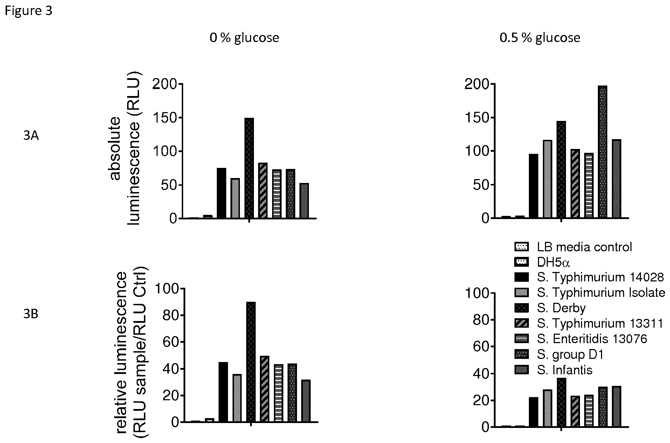

The results are summarized in Figure 3. The quantified signal is presented as relative light units (RLU) in Figure 3A and the normalized signal representing the ratio of the quantified signals obtained from the test sample and from the negative control sample is shown in Figure 3B.

Depending on the *Salmonella* serovar tested, the normalized signal was lower in the presence of 0.5% by weight glucose in the detection medium as compared to detection medium without glucose by a factor of approximately 2. However, even in the presence of glucose, normalized signals in the range from 20 to 40 were obtained. Thus, even though glucose may have a slight negative influence on the performance of the method of the present invention, the method can nevertheless suitably be used for detection of contamination in products for human uptake also in the presence of glucose.

The three examples described above represent exemplary embodiments of the method of the present invention. However, the method may also be performed by other means comprised by the scope of the patent claims. Particularly, detector cells other than *Vibrio harveyi* may be utilized as long as a detectable signal is generated in the detection medium comprising the sample and the respective detector cells. In fact, different detector cells suitable for detection of highly diverse contaminations in products for human uptake are known to the skilled person.

Moreover, the skilled person is well aware of methods for customized generation of detector cells that are suitable for detection of specific contaminations desired to be detected. For example, there are a plenty of different cells, both prokaryotic and eukaryotic, known to the skilled person that comprise contamination receptors interacting with specific contamination-associated molecules. Using standard molecular biology and cell biology methods, the skilled person can easily introduce a detector gene encoding for a suitable detector molecule, like a fluorescent protein or a luciferase protein, into these cells such that the respective detector gene is under the control of a promoter that is activated by interaction of the contamination receptor with the contamination-associated molecule. Respectively modified cells may be used as detector cells according to the present invention.

### Example 4: Correlation between bacterial density and signal strength

It has been reported that the correlation between bacterial density and the concentration of autoinducer molecules might be poor (see Lu et al. in Foodborne Pathogens and Disease, Vol. 2, No. 3, September 2005, pages 242-249). Therefore, it was tested whether the method of the present invention delivers reliable results and allows for conclusions about contamination of products for human uptake from the signal intensity measured.

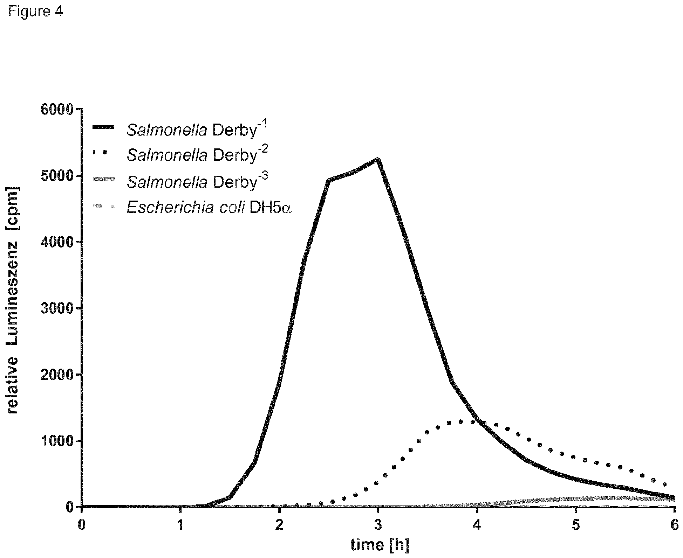

For this reason, different dilutions of *Salmonella* Derby were subjected to the method of the present invention. In detail, a solution of *Salmonella* Derby with a colony forming unit (CFU) of 2.07 x 10⁸/ml was diluted in 10⁻¹ steps and the luminescence of the different samples was compared to the luminescence of a sample of *Escherichia coli* DH5α that served as a negative control. As shown in figure 4, the 10⁻¹-dilution of *Salmonella* Derby showed a strong increase of luminescence after 1.5 hours. The peak of luminescence was reached after about 3 hours at a value of more than 5000 cpm. In contrast, the 10⁻²-dilution of *Salmonella* Derby showed a reasonable increase of luminescence only after 2.5 hours and peaked at about 4 hours at less than 1500 cpm. The 10⁻³-dilution of *Salmonella* Derby did not even reach a relative luminescence of more than a few hundred cpm. Consequently, these experiments suggest that there is a strong positive correlation between bacterial density and strength of the optical signal according to the method of the present invention.

### Example 5: Influence of boric acid on signal strength

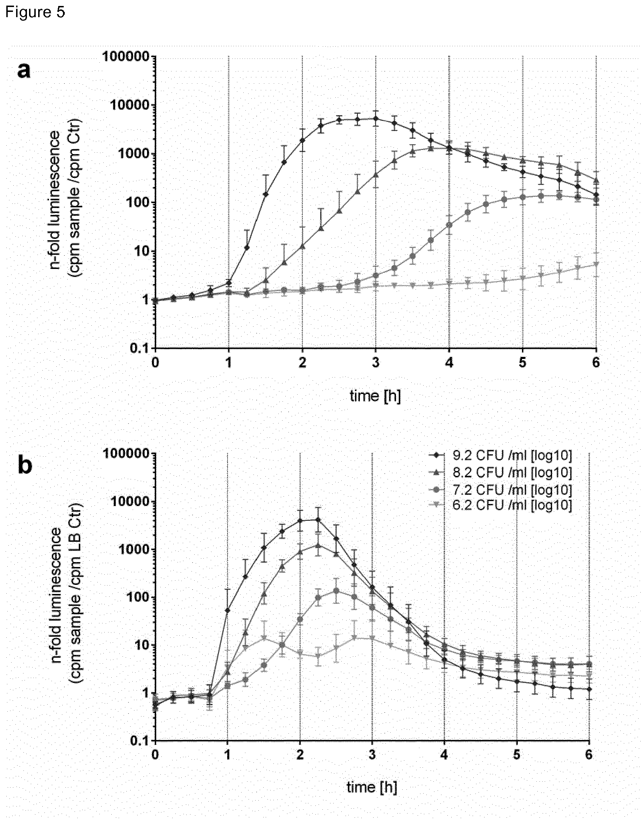

0.25 mM boric acid were added to the liquid detector cell buffer (here AB medium). Figure 5 shows that the addition of 0.25 mM boric acid makes a clear difference in *Salmonella* detection performance. Without addition of boric acid, luminescence induction signals of lower concentrated *Salmonella* cultures were delayed up to 2 hours, while maximal n-fold luminescence induction with addition of boric acid was reached for all *Salmonella* concentrations between 2 and 2.5 hours. The maximal n-fold inductions were similar to the maxima without boric acid. This boric acid dependent effect was also shown for seven other *Salmonella* strains. The lowest concentrated S. Derby 1 culture could only be detected with the addition of boric acid, showing a more than 5-fold luminescence induction after 2 hours (Figure 5).

### Example 6: Co-Detection of different bacterial species

In further experiments it was tested whether co-contamination with different bacterial species had an influence on the luminescence in the method of the present invention. For this reason, *Salmonella* Derby 2002 was separately co-cultured with *Listeria monocytogenes,* and two different strains of *Escherichia coli.* It was found that luminescence was increased by co-cultivation of *Salmonella* Derby 2002 with *Listeria monocytogenes* and also by co-cultivation of *Salmonella* Derby 2002 with *Escherichia coli* K12. Importantly, *Escherichia coli* K12 is a strain that produces the contamination-associated molecule AI-2. Interestingly, co-cultivation of *Salmonella* Derby 2002 with *Escherichia coli* DH5α (a strain that does not produce AI-2) did not result in an increase of luminescence. Also no increase in luminescence was observed when *Salmonella* Derby 2002 was co-cultivated with dead *Listeria monocytogenes.* Thus, the experiments show that contamination with different bacterial species results in higher luminescence in the method of the present invention as long as the bacteria produce a suitable contamination-associated molecule.

### Example 7: Influence of kanamycin

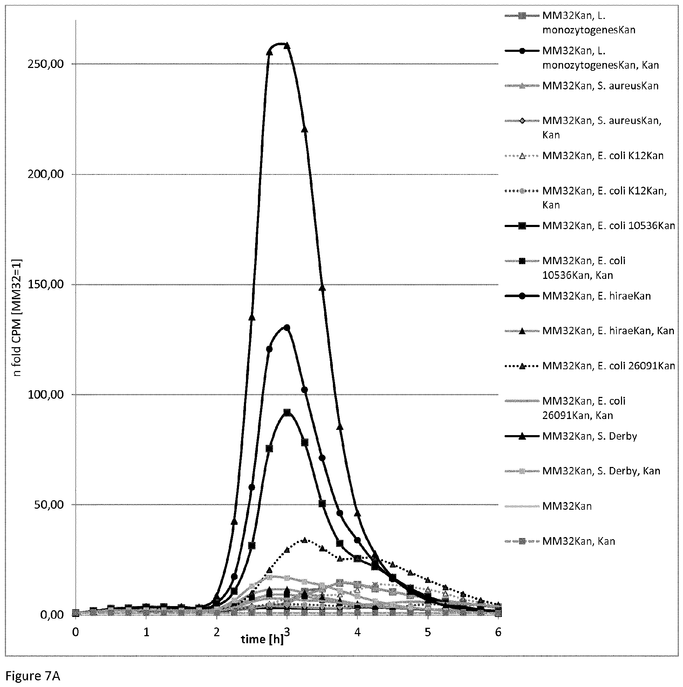

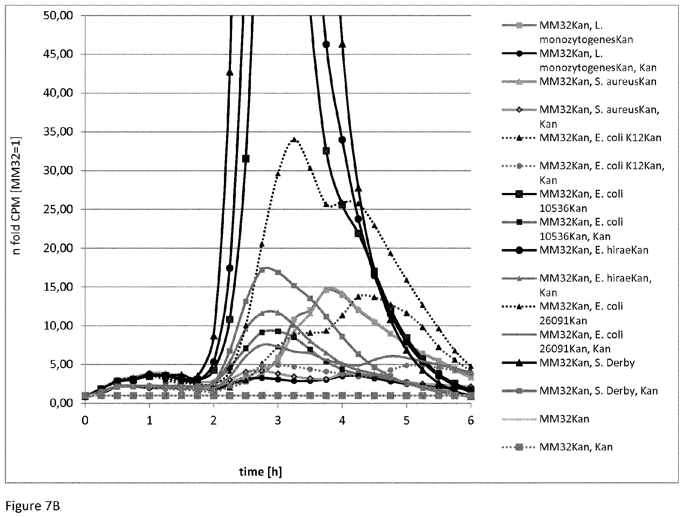

In a last set of experiments, the influence of kanamycin on the method of the present invention was tested. Kanamycin drastically reduced relative luminescence not only with regard to S. Derby but also with regard to other bacterial species as shown in figure 7. Luminescence was reduced even if the tested bacterial species were resistant to kanamycin. The reason is probably that kanamycin partially reduces growth even in resistant bacteria. The experiments show that selective detection of specific bacteria can in principle be achieved with the method of the present invention even if a sample comprises different bacterial species that produce contamination-associated molecules. However, in such a case, a compound has to be added that selectively inhibits growth of specific bacterial species in order to enable a selective distinction.

### Description of the Figures

Figure 1 shows a comparison of results obtained with the method of the present invention utilizing clarified samples or non-clarified samples, respectively. The signal generated in the detection medium was detected after 2 hours (Figure 1A) and 3 hours (Figure 1B) using a microplate reader from Tecan. The normalized signal representing the ratio of the quantified signals obtained from the test sample and from the negative control sample is shown. LB medium without *Salmonellae* was used as negative control sample.
Figure 2 shows a comparison of results obtained with the method of the present invention utilizing non-clarified samples from cultures of different *Salmonella* serovars and of the *E. coli* strain DH5α. The signal generated in the detection medium was detected at the indicated time points using a microplate reader from Tecan. The quantified signal is presented as relative light units (RLU) in Figure 2A and the normalized signal representing the ratio of the quantified signals obtained from the test sample and from the negative control sample is shown in Figure 2B. The time scale represents the time of incubation of the detection medium at the time point of detection of the signal.
Figure 3 shows a comparison of results obtained with the method of the present invention utilizing detection medium without glucose or comprising 0.5% by weight glucose, respectively. The quantified signal is presented as relative light units (RLU) in Figure 3A and the normalized signal representing the ratio of the quantified signals obtained from the test sample and from the negative control sample is shown in Figure 3B.
Figure 4 shows the relative luminescence obtained from different dilutions of *Salmonella* Derby subjected to the method of the present invention. Relative luminescence is shown in count per minute (cpm). *Escherichia coli* DH5α served as a negative control.
Figure 5 shows the effect of boric acid on the sensitivity of the method of the present invention. Different cell concentrations of S. Derby Isolat 1 ovenight cultures were tested. MM32 diluted 1/100 without (a) or with addition of 0.25 mM boric acid (b) served as detector cells. Luminescence induction compared to LB media control that is set to 1 is blotted against time. Error bars indicate standard deviation of three independent experiments.

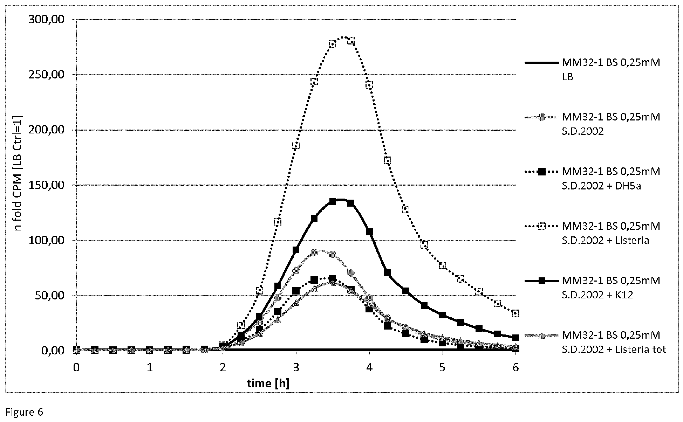

Figure 6 shows the influence of co-contamination with different bacterial species on luminescence in the method of the present invention. *Salmonella* Derby 2002 was co-cultivated with *Listeria monocytogenes* (dead or alive) or with *Escherichia coli* (K12 or DH5α). *Vibrio harveyi* MM32 served as reporter cells. Luminescence is shown relative to MM32 incubated in presence of LB medium without *Salmonella* Derby 2002.
Figure 7 shows the influence of kanamycin on the method of the present invention. Luminescence is shown relative to MM32 incubated in absence of bacterial species to be tested. Kanamycin-resistant species have been indicated as "SpeciesKan", for example "E. coli K12Kan" or "S. aureusKan". Presence of kanamycin in the medium has been indicated by the term "Kan" separated from the listed species by comma. Figure 7B is a zoom into figure 7A.
Figure 8 shows schematically and exemplarily signal generation in detection medium according to an embodiment of the present invention. The detection medium 10 comprises a contamination, for example a pathogen 1, and contamination-associated molecules 2. The detection medium 10 further comprises a detector cell 3, which comprises a contamination receptor 4. In Figure 8, the contamination receptor 4 is shown located at the surface of the detector cell 3. However, the contamination receptor 4 may also be located partially or entirely inside the detector cell 3. A contamination-associated molecule 2 interacts with the contamination receptor 4. The interaction of the contamination receptor 4 with the contamination-associated molecule 2 induces a signal-transduction cascade 5 in the detector cell 3 resulting in increased levels of a detector molecule 6. The detector molecule 6 catalyzes production of signal molecules 7. The signal molecules 7 generate the signal 8.

## Claims

1. A method for detecting contamination in a product comprising the steps of
A. Providing a sample,
B. Providing detector cells, wherein the detector cells comprise at least one contamination receptor, wherein the contamination receptor is a polypeptide,
C. Providing a detection medium comprising the sample and the detector cells,
D. Detecting a signal generated in the detection medium, wherein the generation of the signal is dependent on an interaction of the contamination receptor of the detector cells with a contamination-associated molecule that is present in the product in case of contamination, and
E. Analyzing the signal,
wherein the sample is neither filtered nor centrifuged to remove cells before detecting the signal, wherein the signal is an optical signal, wherein the contamination-associated molecule is selected from the group consisting of autoinducer-1 (AI-1), autoinducer-2 (AI-2), autoinducer-3 (AI-3) and mixtures of two or more thereof, and wherein the detector cells are bacteria.

2. Method according to claim 1, wherein the contamination is a pathogen.

3. Method according to one or more of the preceding claims, wherein the products for human uptake are selected from the group consisting of pharmaceutical products, cosmetic products and food.

4. Method according to one or more of the preceding claims, wherein the sample is selected from the group consisting of non-clarified liquid samples, solid samples and mixtures thereof.

5. Method according to one or more of the preceding claims, wherein the detector cells are provided dispersed in a liquid detector cell buffer as a detector cell dispersion.

6. Method according to one or more of the preceding claims, wherein the intensity of the signal is dependent on an interaction of the contamination receptor with the contamination-associated molecule.

7. Method according to claim 6, wherein the interaction of the contamination receptor with the contamination-associated molecule results in altered levels of at least one detector molecule.

8. Method according to one or more of the preceding claims, wherein the signal is generated by a signal molecule.

9. Method according to one or more of the preceding claims, wherein signal analysis comprises the steps of signal quantification and normalization of the quantified signal.

## Patentansprüche

1. Verfahren zur Detektion von Verunreinigung in einem Produkt, umfassend die Schritte von
A. Bereitstellen einer Probe,
B. Bereitstellen von Detektorzellen, wobei die Detektorzellen mindestens einen Verunreinigungsrezeptor umfassen, wobei der Verunreinigungsrezeptor ein Polypeptid ist,
C. Bereitstellen eines Detektionsmediums, umfassend die Probe und die Detektorzellen,
D. Detektieren eines Signals, erzeugt in dem Detektionsmedium, wobei die Erzeugung des Signals von einer Wechselwirkung des Verunreinigungsrezeptors der Detektorzellen mit einem mit Verunreinigung assoziierten Molekül, das in dem Produkt im Falle von Verunreinigung vorhanden ist, abhängt, und
E. Analysieren des Signals,
wobei die Probe weder filtriert noch zentrifugiert wird, um Zellen vor dem Detektieren des Signals zu entfernen, wobei das Signal ein optisches Signal ist, wobei das mit Verunreinigung assoziierte Molekül aus der Gruppe ausgewählt ist, welche aus Autoinducer-1 (AI-1), Autoinducer-2 (AI-2), Autoinducer-3 (AI-3) und Gemischen von zwei oder mehr davon besteht, und wobei die Detektorzellen Bakterien sind.

2. Verfahren gemäß Anspruch 1, wobei die Verunreinigung ein Pathogen ist.

3. Verfahren gemäß einem oder mehr der vorhergehenden Ansprüche, wobei die Produkte zur Aufnahme durch den Menschen aus der Gruppe ausgewählt sind, welche aus pharmazeutischen Produkten, kosmetischen Produkten und Nahrungsmitteln besteht.

4. Verfahren gemäß einem oder mehr der vorhergehenden Ansprüche, wobei die Probe aus der Gruppe ausgewählt ist, welche aus nicht-geklärten flüssigen Proben, festen Proben und Gemischen davon besteht.

5. Verfahren gemäß einem oder mehr der vorhergehenden Ansprüche, wobei die Detektorzellen dispergiert in einem flüssigen Detektorzellpuffer als eine Detektorzelldispersion bereitgestellt werden.

6. Verfahren gemäß einem oder mehr der vorhergehenden Ansprüche, wobei die Intensität des Signals von einer Wechselwirkung des Verunreinigungsrezeptors mit dem mit Verunreinigung assoziierten Molekül abhängt.

7. Verfahren gemäß Anspruch 6, wobei die Wechselwirkung des Verunreinigungsrezeptors mit dem mit Verunreinigung assoziierten Molekül in veränderten Mengen von mindestens einem Detektormolekül resultiert.

8. Verfahren gemäß einem oder mehr der vorhergehenden Ansprüche, wobei das Signal durch ein Signalmolekül erzeugt wird.

9. Verfahren gemäß einem oder mehr der vorhergehenden Ansprüche, wobei Signalanalyse die Schritte von Signalquantifizierung und Normalisierung des quantifizierten Signals umfasst.

## Revendications

1. Procédé de détection d'une contamination dans un produit, comprenant les étapes suivantes :
A. une fourniture d'un échantillon,
B. une fourniture de cellules de détection, dans laquelle les cellules de détection comprennent au moins un récepteur de contamination, dans laquelle le récepteur de contamination est un polypeptide,
C. une fourniture d'un milieu de détection comprenant l'échantillon et les cellules de détection,
D. une détection d'un signal produit dans le milieu de détection, dans laquelle la production du signal dépend d'une interaction du récepteur de contamination des cellules de détection avec une molécule associée à la contamination présente dans le produit en cas de contamination, et
E. une analyse du signal,
dans lequel l'échantillon n'est ni filtré ni centrifugé pour éliminer les cellules avant la détection du signal, dans lequel le signal est un signal optique, dans lequel la molécule associée à la contamination est choisie dans le groupe constitué de l'auto-inducteur-1 (AI-1), de l'auto-inducteur-2 (AI-2), de l'auto-inducteur-3 (AI-3), et de mélanges de deux ou plus de ceux-ci, et dans lequel les cellules de détection sont des bactéries.

2. Procédé selon la revendication 1, dans lequel la contamination est un agent pathogène.

3. Procédé selon une ou plusieurs des revendications précédentes, dans lequel les produits destinés à une consommation humaine sont choisis dans le groupe constitué des produits pharmaceutiques, des produits cosmétiques et de l'alimentation.

4. Procédé selon une ou plusieurs des revendications précédentes, dans lequel l'échantillon est choisi dans le groupe constitué des échantillons liquides non clarifiés, des échantillons solides et des mélanges de ceux-ci.

5. Procédé selon une ou plusieurs des revendications précédentes, dans lequel les cellules de détection sont fournies de façon dispersée dans un tampon liquide de cellules de détection en tant que dispersion de cellules de détection.

6. Procédé selon une ou plusieurs des revendications précédentes, dans lequel l'intensité du signal dépend d'une interaction du récepteur de contamination avec la molécule associée à la contamination.

7. Procédé selon la revendication 6, dans lequel l'interaction du récepteur de contamination avec la molécule associée à la contamination a pour effet des niveaux altérés d'au moins une molécule de détection.

8. Procédé selon une ou plusieurs des revendications précédentes, dans lequel le signal est produit par une molécule signal.

9. Procédé selon une ou plusieurs des revendications précédentes, dans lequel l'analyse du signal comprend les étapes de quantification du signal et de normalisation du signal quantifié.
